# EUROPEAN PATENT SPECIFICATION

(11) **EP 4 100 418 B1**
(45) Date of publication and mention of the grant of the patent: **08.07.2026**
(21) Application number: 21703433.9
(22) Date of filing: 04.02.2021
(51) Int. Cl.: C07K 1/12, C07K 2/00, C08F 226/00, B01J 31/08, A23J 3/32, A23K 20/142, A23K 20/147, A23K 50/10, A23K 50/30, A23K 50/40, A23K 50/75, A23K 50/80, A23L 33/18, A23L 33/175, C08F 226/06

(54) **USE OF IONIC POLYMERS FOR HYDROLYSIS OF PROTEINS AND PROTEIN-CONTAINING FEEDSTOCK**
VERWENDUNG VON IONISCHEN POLYMEREN ZUR HYDROLYSE VON PROTEINEN UND PROTEINHALTIGEN EINSATZSTOFFEN
UTILISATION DE POLYMÈRES IONIQUES POUR L'HYDROLYSE DE PROTÉINES ET CHARGE CONTENANT DES PROTÉINES

(30) Priority: 04.02.2020 EP 20155416
(43) Date of publication of application: 14.12.2022
(73) Proprietor: Embion Technologies SA, 1024 Ecublens (CH)
(72) Inventor: SIANKEVICH, Sviatlana, 1173 Féchy (CH); SAVOGLIDIS, Georgios, 1173 Féchy (CH); DYSON, Paul, 1024 Ecublens (CH); BÄNSCH, Johannes, 1024 Ecublens (CH)
(74) Representative: KATZAROV S.A.
(86) International application number: PCT/EP2021/052597
(87) International publication number: WO 2021/156331

(56) References cited:
- WO-A1-2019/058270
- XU XUEJIAO ET AL: "Trypsin entrapped in poly(diallyldimethylammonium chloride) silica sol-gel microreactor coupled to matrix-assisted laser desorption/ionization time-of-flight mass spectrometry", RAPID COMMUNICATIONS IN MASS SPECTROMETRY, vol. 22, no. 8, 27 March 2008 (2008-03-27), GB, pages 1257 - 1264, XP055798657, ISSN: 0951-4198, DOI: 10.1002/rcm.3478
- HYUN CHANG-KEE ET AL: "Utilization of bovine blood plasma proteins for the production of angiotensin I converting enzyme inhibitory peptides", PROCESS BIOCHEMISTRY, vol. 36, no. 1-2, 14 September 2000 (2000-09-14), GB, pages 65 - 71, XP055799221, ISSN: 1359-5113, DOI: 10.1016/S0032-9592(00)00176-X

## Description

### FIELD OF THE INVENTION

The invention relates to use of ionic polymers (IP) consisting of anions and a polymeric backbone containing cations, in hydrolyzing proteins and protein-containing feedstock to produce protein hydrolysate.

### BACKGROUND OF THE INVENTION

Proteins make up all of the body's organs and are required for proper function of organ systems. All proteins are made up of amino acids, but differences in amino acid composition and sequence differentiate how proteins function. The body uses amino acids to construct specific proteins for specific functions in the maintenance of organ health. However, the body has no de novo route for synthesis of many necessary amino acids, therefore these essential amino acids must be obtained from dietary protein. The body must consistently digest, absorb and metabolize adequate dietary proteins to supply organs with the specific proteins needed to function.

Dietary protein can be more easily digested and absorbed by consuming smaller peptide subunits of proteins or even a mixture of amino acids. Peptides and amino acids can be obtained by hydrolyzing proteins that often come from plant, animal, fungal or microorganism sources. Peptides and amino acids are usually obtained by acidic, alkaline or enzymatic hydrolysis of proteins. Alkaline hydrolysis process results in the complete destruction of most amino acids (almost 100% loss). Acid hydrolysis process offers the advantage of low cost. However, this process results in the complete destruction of tryptophan, a partial loss of methionine, and the conversion of glutamine into glutamate and of asparagine into aspartate. The main disadvantages of enzymatic hydrolysis of proteins is that pH and temperature ranges that optimize enzyme activity tend to maximize protein folding. High stearic hindrance results in poorly hydrolysed proteins, which adversely affects solubility, absorption, potency, sensory properties and interaction stability. Furthermore, enzymatic hydrolysis of protein includes the relatively high cost and the potential presence of enzyme inhibitors in the raw protein materials.

Industrial processes for hydrolysing proteins to produce protein hydrolysates comprising oligopeptides, peptides and/or amino acids rely mainly on enzyme mixtures which are far from optimal so that expensive purification steps are needed to produce peptide mixtures having sub-optimal size distributions.

XU XUEJIAO et al., "Trypsin entrapped in poly( diallyldimethylammonium chloride) silica sol-gel microreactor coupled to matrix-assisted laser desorption/ionization time-of-flight mass spectrometry", RAPID COMMUNICATIONS IN MASS SPECTROMETRY, vol. 22, no. 8, 27 March 2008, p.1257-1264, discloses a method for hydrolysing Bovine Serum Albumin (BSA) protein into digested peptides.

WO 2019/058270 A1 discloses ionic polymers used as a catalyst to degrade a biomass into a liquid- and a solid phase, in order to recover fine chemicals from said liquid phase.

Therefore, there is still a need for simple and safe method for hydrolysing proteins from various sources to produce protein hydrolysates of high digestibility, high absorption properties and high benefits.

### SUMMARY OF THE INVENTION

An aspect of the present invention provides a method for hydrolysing proteins and/or protein-containing feedstock into protein hydrolysate, the method comprising the steps of:
a) providing protein and/or protein-containing feedstock;
b) optionally determining protein content in the protein-containing feedstock;
c) optionally pre-treating the protein-containing feedstock;
d) optionally isolating proteins from the protein-containing feedstock to form a protein concentrate;
e) contacting the protein, the protein concentrate and/or the protein-containing feedstock with a catalyst to form a reaction mixture, wherein the catalyst is an ionic polymer, the ionic polymer network, a solid-supported ionic polymers and/or a polymer membrane incorporating ionic polymers;
f) degrading the protein, the protein concentrate and/or the protein-containing feedstock in the reaction mixture to produce a liquid phase and a solid phase, wherein the liquid phase includes a protein hydrolysate, and the solid phase includes residual materials;
g) isolating at least a portion of the liquid phase from the solid phase; and
h) recovering the protein hydrolysate from the isolated liquid phase;
wherein the ionic polymer (IP) is 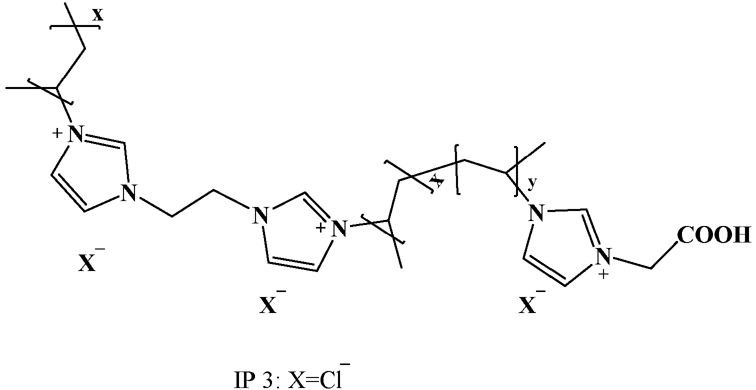
wherein x and y are integers each independently selected within the range 1 to 1000;
wherein the ionic polymer network comprises cross-linked the one or more ionic polymers (IP);
wherein the solid support has at least one surface comprising the one or more ionic polymers (IP) or the ionic polymer network;
wherein the polymer membrane incorporates the one or more ionic polymers (IP) or the ionic polymer network.

Another aspect of the present invention provides the protein hydrolysate obtained by the method for hydrolysing proteins and/or protein-containing feedstock of the present invention.

Another aspect of the present invention provides a use of the protein hydrolysate of the present invention in the manufacturing of food products, animal feed products and cosmetic products.

Another aspect of the present invention provides a food product comprising an edible material and the protein hydrolysate of the present invention.

### BRIEF DESCRIPTION OF THE FIGURES

**Figure 1** shows MS(+) chromatogram of the hydrolysed gluten mixture (top) and generated peptides profile (bottom).
**Figure** 2 shows MS(+) chromatogram of the hydrolysed albumin mixture.
**Figure** 3 shows MS(+) chromatogram of the hydrolysed spent barley mixture.
**Figure 4** shows growth of a *Lactobacillus X* strain in the presence of Glucose, FOS, XOS and the composition Prembion comprising protein hydrolysate of the invention. Colony counts as a function of growth time for a *Lactobacillus X* strain that has been reported to be responsible, in part, for human and animal gut health. All additives were added on a 1% (by total sugars basis) level.
**Figure** 5 shows growth of a *Bifidobacterium X* strain in the presence of Glucose, FOS, XOS and the composition Prembion comprising protein hydrolysate of the invention. Colony counts as a function of growth time for a *Bifidobacterium X* strain that has been reported to be responsible, in part, for human and animal gut health. All additives were added on a 1% (by total sugars basis) level.

### DETAILED DESCRIPTION OF THE INVENTION

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components. Also as used in the specification and claims, the language "comprising" can include analogous embodiments described in terms of "consisting of " and/or "consisting essentially of".

As used in the specification and claims, the singular form "a", "an" and "the" include plural references unless the context clearly dictates otherwise.

As used in the specification and claims, the term "and/or" used in a phrase such as "A and/or B" herein is intended to include "A and B", "A or B", "A", and "B".

The term "monomer" refers to a molecule that can undergo polymerization or copolymerization thereby contributing constitutional units to the essential structure of a macromolecule (a polymer).

"Cross-linking", as used herein, refers to the attachment of two or more monomers, oligomers or longer polymer chains by bridges of a cross-linker, such as an element, molecular group, a compound, or another oligomer or polymer. Cross-linking can result in a polymeric network (which can be two-dimensional or three-dimensional) where the polymer subunits are interconnected with multiple cross-linking agents and without free ends. Cross-linking may take place upon exposure to a stimulus, such as heat or light. As a result, some cross-linking processes occur at increased temperature, and some may also occur at room temperature or at lower temperature. As cross-linking density is increased, the properties of a material can be changed from thermoplastic to thermosetting.

The terms "peptide" or "oligopeptide", as used herein, are defined as a chain of at least two amino acids that are linked through peptide bonds. The terms "peptide" and "oligopeptide" can be used interchangeably as the context requires.

A protein consists of one or more chain comprising of more than 30 amino acid residues (polypeptides) linked together by peptide bonds.

As used herein a "protein hydrolysate" (or "hydrolysate" or "hydrolysed protein") is the product that is formed by hydrolysis of the protein peptide bonds between amino acids and it refers to a mixture of amino acids and peptides of different chain length. An enriched hydrolysate being a fraction of the protein hydrolysate, for example enriched in selected peptides or wherein a subset of peptides or polypeptides have been removed from the hydrolysate. So an enriched hydrolysate is preferably a mixture of peptides or a peptide mixture.

As used herein, the term "food product" refers to any food or feed suitable for consumption by humans, non-ruminant animals, or ruminant animals. The "food product" may be a prepared and packaged food (e.g., mayonnaise, salad dressing, bread, or cheese food) or an animal feed (e.g., extruded and pelleted animal feed or coarse mixed feed).

An aspect of the present invention provides a method for hydrolysing proteins and/or protein-containing feedstock into protein hydrolysate, as defined in the appended claims.

In an embodiment, the present invention provides a method for hydrolysing proteins and/or protein-containing feedstock into a protein hydrolysate, the method comprising the steps of:
a) providing protein and/or protein-containing feedstock;
b) optionally determining protein content in the protein-containing feedstock;
c) optionally pre-treating the protein-containing feedstock;
d) optionally isolating proteins from the protein-containing feedstock to form a protein concentrate;
e) contacting the protein, the protein concentrate and/or the protein-containing feedstock with a catalyst to form a reaction mixture, wherein the catalyst is an ionic polymer, the ionic polymer network, a solid-supported ionic polymers and/or a polymer membrane incorporating ionic polymers;
f) degrading the protein, the protein concentrate and/or the protein-containing feedstock in the reaction mixture to produce a liquid phase and a solid phase, wherein the liquid phase includes the protein hydrolysate, and the solid phase includes residual materials;
g) isolating at least a portion of the liquid phase from the solid phase; and
h) recovering the protein hydrolysate from the isolated liquid phase;
wherein the ionic polymer (IP) is 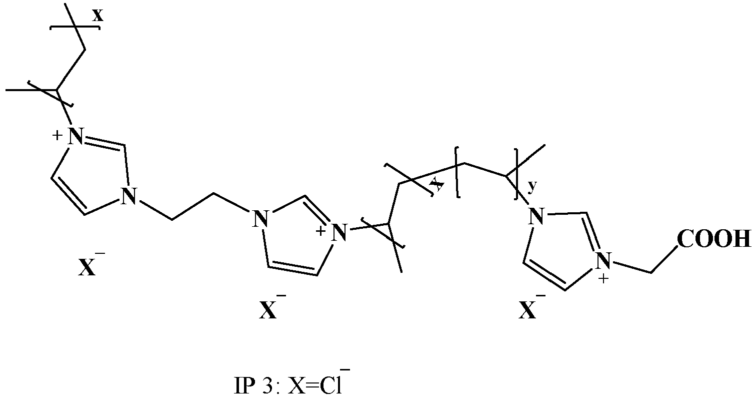
wherein x and y are integers each independently selected within the range 1 to 1000;
wherein the ionic polymer network comprises cross-linked the one or more ionic polymers (IP);
wherein the solid support has at least one surface comprising the one or more ionic polymers (IP) or the ionic polymer network;
wherein the polymer membrane incorporates the one or more ionic polymers (IP) or the ionic polymer network.

In one embodiment, the step e) contacting the protein, the protein concentrate and/or the protein-containing feedstock with a catalyst to form a reaction mixture consists in adding water or an appropriate organic solvent and an effective amount of the catalyst to the protein, the protein concentrate and/or the protein-containing feedstock to form a reaction mixture, wherein the catalyst is an ionic polymer of the invention or a combination of ionic polymers of the invention, the ionic polymer network of the invention, a membrane incorporating ionic polymers of the invention and/or a solid-supported ionic polymers of the invention; and degrading step f) consists in heating the reaction mixture of step e) during appropriate time and subsequently cooling to room temperature (typically 20-25 °C). In some embodiments, the heating is between 50° to 170°C or 100°C to 160°C or 110°C to 150°C or 140°C to 160°C or 100°C to 170°C. In some other embodiments heating is at maximum 50°C or at maximum 100°C, or at maximum 170°C. In other embodiments, the appropriate time is typically 0.5 to 3 hours. The reaction temperature and time depend on the origin of the protein or protein-containing feedstock.

In some embodiments of the method for hydrolysing proteins and protein-containing feedstock into protein hydrolysate of the present invention, the method further comprises applying a pressure of N₂ or CO₂ during the degrading step f). The pressure may range from 20 bar to 300 bar, preferably from 20 to 150 bar.

The term "protein", as used herein, refers to any animal, plant and microorganism protein.

The term "protein-containing feedstock", as used herein, refers to living or dead biological material that can be used in the method for producing the protein hydrolysate of the present invention. In some embodiments, the protein-containing feedstock is selected from the group comprising plant-based protein, single-cell protein, in vitro meat, yeast extract, spent yeast or yeast slurry, spent barley, insects, soybeans, pea, rapeseed, whey, casein, wheat, canola, corn, Jatropha, palm, peanut, sunflower, coconut, mustard, cotton seed, Palm kernel, olive, safflower, sesame, linseed, algae, crustaceans, fish meal, meat and bone meal, molasses, sprouted grains and legumes, collagen and other main commercial categories of protein isolates.

Optionally, prior to any use, protein content is determined in the protein-containing feedstock according the Bradford protein assay, Kjeldahl's method or Lowry method.

The optional pre-treatment of the protein-containing feedstock, used in the methods described herein, uses one or more methods selected from the group consisting of washing, solvent-extraction, solvent-swelling, comminution, milling, steam pre-treatment, explosive steam pre-treatment, dilute acid pre-treatment, hot water pre-treatment, alkaline pre-treatment, lime pre-treatment, wet oxidation, wet explosion, ammonia fibre explosion, organosolvent pre-treatment, biological pre-treatment, ammonia percolation, ultrasound, electroporation, microwave, supercritical CO₂, supercritical H₂O, ozone, and gamma irradiation. The optional pre-treatment of the protein-containing feedstock includes for example the milling of the protein-containing feedstock.

The optional isolating proteins from the protein-containing feedstock to form a protein concentrate, used in the method described herein, uses one or more selective precipitation methods selected from the group consisting of salting out; isoionic precipitation; organic co-solvent precipitation; two carbon (C₂) organic co-solvent precipitation of proteins; C₄ and C₅ organic co-solvent precipitation, phase partitioning and extraction of proteins; protein exclusion and crowding agents (neutral polymers) and osmolytes; synthetic and semisynthetic polyelectrolyte precipitation; metallic and polyphenolic heteropolyanion precipitation; hydrophobic ion pairing (HIP) entanglement ligands; matrix-stacking ligand co-precipitation; di- and trivalent metal cation precipitation.

Some ionic polymers used in the method of the invention for hydrolysing proteins and protein-containing feedstock into protein hydrolysate consists of anions and a polymeric backbone containing cations as disclosed in WO 2019/058270 A1.

Ionic polymers (IPs) of the present disclosure can be synthesized via several methods, including but not limited to the direct polymerization of appropriate ionic species, the chemical modification of non-IPs, etc. in different solvents (water, acetonitrile, alcohols (methanol, ethanol, propanol etc.), toluene, THF) (see Examples). Polymerization may include different approaches, e.g. free radical polymerization, living/controlling radical polymerization, reversible addition-fragmentation transfer, ionic and coordination polymerization. The anionic structure can be designed according to preference before or after polymerization. The resulting ionic polymer (IP) combines the general properties of the ionic monomer and the enabling properties of a solid catalyst due to the presence of specific functional groups. In an embodiment of the present disclosure, a salt is prepared with a cation and an anion, wherein both the cation and the anion contain vinyl groups that can be polymerized using AIBN or other initiator. It is essentially a very simple method and the ionic polymer is purified by removal of the excess AIBN by washing and filtration. In a specific embodiment of the present disclosure, a salt that is composed of the 1-(1-vinylimidazolium)ethyl-3-vinylimdazolium] [dichloride]) is prepared. This salt, a pure compound, is then polymerized using the radical initiator AIBN. The ionic polymer is purified by removal of the excess AIBN by washing and filtration. As alternative to dichoride anion, a ditriflate anion can be obtained via anion exchange reaction prior polymerization.

In some embodiments, the ionic polymer network further comprises itaconic acid, citric acid and/or 1,4 butanediol.

In other embodiments, the ionic polymer network further comprises one or more metal catalysts. In some embodiments, the metal catalyst is a metal salt. In preferred embodiments anion in metal salt is selected from the group comprising F⁻, Cl⁻, Br⁻, I⁻, ClO₄⁻, BF₄⁻, PF₆⁻, AsF₆⁻, SbF₆⁻, NO₂⁻, NO₃⁻, HSO₄⁻, SO₄²⁻, PO₄³⁻, HPO₄²⁻, CF₃CO₂⁻, CF₃CO₃⁻, CO₃²⁻, CF₃SO₃⁻, C₁-C₆ carboxylate, CN⁻, SCN⁻, OCN⁻, CNO⁻, N₃⁻, tosylate, mesylate, trifluoromethanesulfonate, trifluoroethane sulfonate, di-trifluoromethanesulfonyl amino, docusate, xylenesulfonate salts, and metal ion is selected from the group comprising Na, Ba, Sr, Ca, Cd, Sn, Pb, Fe, Cu, Zn, Zr, Mn, Co, Ni, Li, Al, Cr, Mg, Mo, Hg, Ag, Au, Pt, Rh, Re, Ti, Pb, Bi, Ga, In, Sn, Ir, La, Hf, Ta, W, Os.

In some preferred embodiments, C₁-C₆ carboxylate are selected from the group comprising formate, acetate, propionate, butyrate, hexanoate, maleate, fumarate, oxalate, lactate, pyruvate.

The ionic polymer network comprising one or more metal catalysts provides better stability and reusability of the ionic polymer-metal combinations.

The preparation of the ionic polymer network with one or more metal catalysts typically consists in mixing or refluxing the ionic polymer network and metal salt in water/organic solvent overnight. See for example J. Am. Chem. Soc., 2012, 134, 11852-11855; Chem. Cat. Chem., 2016, 8, 2508 -2515; J. Org. Chem., 2011, 76 (24), pp 10140-10147; Inorg. Chem., 2006, 45, 6396-6403.

The ionic polymers can be incorporated in membranes or attached to solid supports.

In some embodiments, a polymer membrane comprises one or more ionic polymers. By adding appropriate copolymer (for example acrylic acid) to the salt used for preparation of ionic polymers and then polymerize the mixture it is possible to generate a polymer membrane. An approach for membrane formation is based on the template-free method via simple ionic complexations when an ionic monomer is copolymerized with appropriate organic acid/acid derivative (see Täuber K. et al, Polym. Chem., 2015, 6, 4855-4858; Täuber K. et al, ACS Macro Lett., 2015, 4(1), 39-42; Zhang S. et al, Chem. Sci., 2015, 6, 3684-3691). As example, ionic monomer was dissolved in DMSO and stirred for 2 h at 60 °C. The transparent solution was then poured onto a glass plate and the solvent was evaporated at 80 °C in an oven. The resulting non-porous dry polymer film was subsequently immersed into aqueous ammonia (0.2 wt%) overnight for pore formation and electrostatic complexation. The membrane was detached easily from the glass plate and washed several times with water.

In some embodiments, a solid support has at least one surface comprising one or more ionic polymers. Supported ionic polymers can be immobilized on different materials as a support: silicon or carbon (nanotube, wire) source, graphene or graphene oxide, zeolites, metal/metals alloys or metal/metal alloy oxides. As example, FeOₓ support has been oxidized in the oven in presence of oxygen at high temperature (500 °C) and its surface was modified with mixture of silanes dissolved in ethanol in presence of HCl afterwards. After drying at room temperature, the support was uniformly impregnated with methanol solution of ionic polymer and AIBN. After drying at room temperature, the obtained material was placed in the oven at 95 °C for 2 h. By repeating the impregnation process the desire polymer loading might be achieved. Another example is stainless steel membrane comprising ionic polymers. A mixture containing ionic monomer (0.2-0.5, molar ratio), acrylic acid (0.1-0.6, molar ratio), and benzoin ethylether (1 wt%, as a photo-initiator) were dissolved in methanol to achieve a homogeneous solution, which was then dispersed by wettening onto stainless steel membrane and photo-crosslinked at room temperature by irradiation with UV light of 250 nm wavelength. Ionic polymer attachment is also possible through surface grafting, which requires activation of the support by UV or O₃, O₂, H₂ or air plasmas. It involves the creation of reactive sites (radicals) on the polymer surface followed by the covalent linkage of a preformed polymer or, more commonly, by the polymerization of a monomer from those radical sites (see Alves P. et al, Colloids and Surfaces B: Biointerfaces, Volume 82, Issue 2, 1 February 2011, 371-377; Barbey R. et al., Chem. Rev., 2009, 109(11), 5437-5527). Another copolymer or polymerization initiator might also be used during the polymerisation process (as in case of membrane formation).

In some embodiments of the method for hydrolysing proteins and protein-containing feedstock into protein hydrolysate of the present invention, the organic solvent is selected from the group comprising alcohol (such as methanol, ethanol, butanol, ethylene glycol, etc.), ether (such as dimethoxyethane, diglyme, butyl methyl ether, etc.), ketone (such as methyl isobutyl ketone, N-methyl-2-pyrrolidone, etc.), eutectic solvents (such as glycerol - choline chloride, octanoic acid - tetrabutylammonium chloride, poly (ethylene glycol) - choline chloride, lactic acid - glycine).

In some embodiments of the methods of the present invention, recovering the protein hydrolysate can be done by any technic known in the art, such as filtration, centrifugation or gravity settling.

The effective amount of the ionic polymers or a combination thereof used in the methods described herein can depend on several factors including, for example, the type of the protein or the protein-containing feedstock, the amount of the protein-containing feedstock, the content of proteins in the protein-containing feedstock, the type and number of pre-treatment(s) applied to the protein-containing feedstock, and the reaction conditions (such as temperature and time). An effective amount of the ionic polymer refers to an amount sufficient to degrade the proteins or the protein-containing feedstock into the protein hydrolysate of the invention. In some embodiments, the effective amount of the ionic polymer of the invention is usually 0.05:1 w/w to 10:1 w/w, 0.5:1 w/w to 10:1 w/w, 1:1 w/w to 1:5 w/w, preferably 0.1:1 w/w to 1:5 w/w compared to protein content in the protein-containing feedstock.

The ratio protein-containing feedstock to water used in the methods described herein can depend on several factors, including for example the type of the protein-containing feedstock and the amount of the protein-containing feedstock. In some embodiments, the ratio the protein-containing feedstock to water or organic solvent (such as alcohol, ether, ketone, eutectic solvent) used in the methods described herein is ranging from 1:100 w/v to 1:1 w/v, preferably 1:50 w/v to 1:10 w/v.

The preferred temperature profile for the heating used in the methods described herein depends on the protein-containing feedstock starting material being used and also the intended protein hydrolysate being produced. The heating temperature should preferably be held at a maximum of 200°C, in some embodiments at a maximum of 170°C or 160°C. In some embodiments, the heating temperature is between 100°C and 200°C, or between 100°C and 170°, or between 100°C and 160°C, or between 110°C and 150°C; preferably between 120°C to 170°C or between 120°C to 140°C. Preferably, for small-scale applications, the heating is done in a highpressure autoclave reactor, which after sealing, is heated for appropriate reaction time and temperature.

The method for producing the protein hydrolysate of the present invention operates at moderate temperatures, typically less than 170°C or 160°C or even less than 50°C, whereas the prior art methods needs temperatures of more than 170°C. In addition, the method for producing the protein hydrolysate of the present invention provides fewer by-products, which allows easier recovery of the desired products.

In some embodiments, the appropriate reaction time in the methods described herein is for example between 10 minutes and 10 hours, preferably between 0.5 hour and 5 hours or between 1 hour and 3 hours, depending on the type and amount of the protein-containing feedstock.

An important advantage of the ionic polymers, membranes incorporating ionic polymers and/or solid-supported ionic polymers and use thereof for protein and protein-containing feedstock hydrolysis, decomposition or degradation is their use in one-pot systems for decomposition and selective extracting the aforementioned protein hydrolysate from the protein-containing feedstock. Further, ionic polymers of the present disclosure are insoluble, thus do not mix with the protein hydrolysate of the present invention. According to the present invention, to make proteins a more appealing food substitute having improved digestibility, absorption and benefits, the large native proteins are hydrolysed into smaller protein fragments called protein hydrolysates, such as peptides and/or amino acids, with ionic polymers disclosed herein.

Another aspect of the present invention provides a protein hydrolysate obtained by the method for hydrolysing proteins and/or protein-containing feedstock of the present invention. In some embodiments, the protein hydrolysate obtained by the method of the invention comprises peptides and free amino acids, wherein the protein hydrolysate is water soluble, having improved dissolving properties and degree of hydrolysis above 2.5% and wherein peptides are oligopeptides and polypeptides, optionally linked to carbohydrates, having a molecular weight of less than 10'000 Da.

In some embodiments, the oligopeptides and/or the polypeptides in the protein hydrolysate have a molecular weight of less than 10'000 Da, preferably of 500 to 8000 Da.

The peptides typically contain 2 to 20 amino acids or more. The peptides can be bioactive. The protein hydrolysate of the invention is water soluble, having improved dissolving properties and degree of hydrolysis above 2.5%

The protein hydrolysate of the present invention can act as physiological modulators of metabolism and can possess a wide range of bioactivities including immunomodulatory, anticancer, antihypertensive, antioxidant, anti-inflammatory, mineral binding, opiate, antilipemic, antimicrobial / antibacterial, antifungal and antiviral agents, anticoagulants, thermogenic agents, anti-osteoporosis (osteoprotective), cell growth and repair modulators, angiotensin-converting enzyme (ACE) inhibitors, in addition to biological signalling mediators involved in a myriad of signalling functions with impact on recovery, lipid metabolism, carbohydrate metabolism, immune function, cardiovascular and bone health, nervous system and brain function, optimizing muscle performance during exercise, digestive satiety and weight management.

**In** addition to bioactivities, the protein hydrolysate of the present invention possess various physicochemical properties including solubility, lipid binding, foaming, and emulsification properties depending on their composition, sequence, and length. Indeed, the protein hydrolysate of the present invention can exert beneficial effects on improving intestinal morphology, function, and resistance to infectious diseases in humans and animals (such as pigs, calves, chickens, companion animals and fish), thereby enhancing their health and well-being, as well as growth performance and feed efficiency. This provides a cost-effective approach to converting protein-containing feedstock, such as animal by-products, brewer's by-products, or plant feedstuffs, into high-quality protein-hydrolysate ingredients to provide human dietary supplements and animal feed formulations. Further, the protein hydrolysate of the present invention can have applications in textiles including applications such as plywood adhesives; aquaculture and agriculture including applications such as promotion of plant rooting, germination, growth and prolong lifetime; cosmetic formulation; biopharmaceuticals and absorbent hydrogel formulation; functional food and beverage formulation; enteric diet formulation and dietary supplementation; infant and paediatric nutritional product formulation; animal feed formulation; cell culture growth medium and fermentation processing; baking ingredient to improve resistance against freezing and favourable texture.

The protein hydrolysate of the present invention can be used in various applications selected from the group comprising pharmaceutical, preventative health, dietary supplement, functional food and beverage, paediatric nutrition, food additive, animal feed, fertilizer, antioxidant, antimicrobial, cosmetic, surfactant applications.

Preferably, the protein hydrolysate of the present invention is used in the manufacturing of a food product, a functional food, a nutritional supplement, an animal feed product and/or cosmetic product.

In another embodiment, the present invention provides a use of the protein hydrolysate of the present invention in the manufacturing of food products, animal feed products and/or cosmetic products.

Another aspect of the present invention provides a food product comprising the protein hydrolysate of the present invention and an edible material. In the present invention, the food product comprises an edible material and the protein hydrolysate of the present invention. The selection of a particular protein hydrolysate to combine with an edible material can and will vary depending upon the desired food product. The selection of the appropriate edible material also will vary depending on the desired food product. The edible material may be a plant-derived material (for example a vegetable juice, a cereal product, etc.), an animal-derived material (for example a dairy product, an egg product, etc.), or a biomaterial (for example a protein, a carbohydrate, a lipid, etc.) isolated from a plant-derived material or an animal-derived material, and so forth.

The food product of the present invention may include, for example, hot or cold cereals, bars, baked goods, beverages, yogurts, desserts, snacks, pastas, and meats (including poultry and seafood).

The protein hydrolysate of the present invention can be combined with all kinds of ingredients such as oils, fats, emulsifiers, carbohydrates, fruit concentrates, flavours, colorants, alcohol, carbon dioxide, thickeners, acidulates, antioxidants, herbs or herb extracts, health promoting compounds like vitamins or bioactive compounds to formulate a product which is in line with the marketing needs.

The foregoing description will be more fully understood with reference to the following Examples. Such Examples, are, however, exemplary of methods of practising the present invention and are not intended to limit the application and the scope of the invention.

### EXAMPLES

### Preparation of protein hydrolysate from whey gluten

This example is not according to the invention and is present for illustration purposes only. 5g of whey gluten suspended in 75 ml of water together with 65 mg of **IP1** were stirred at 140°C for 1h. After reaction, the mixture was cooled to room temperature, filtered, solid phase was dried and the liquid phase was subjected for QTOF-ESI(+) analysis. 35% weight conversion was measured.

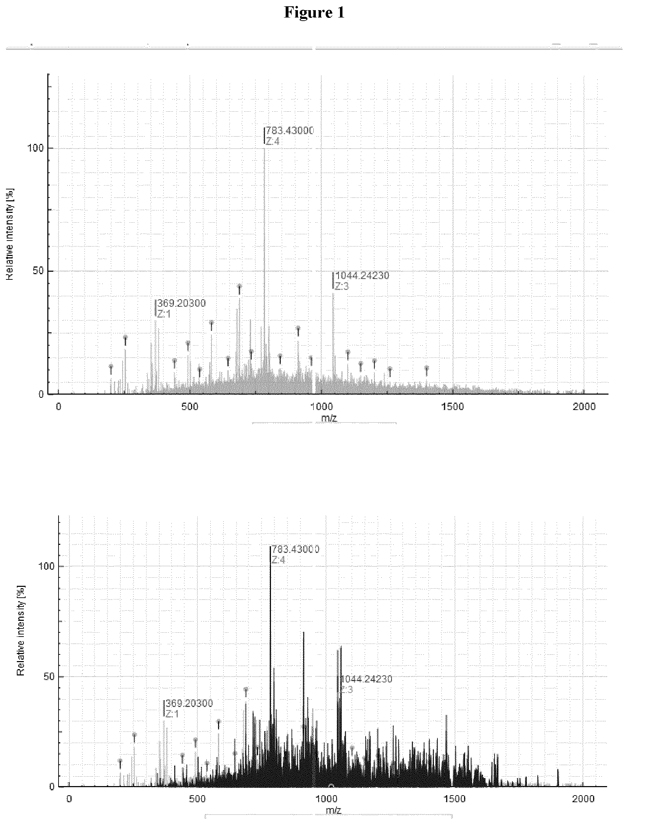

The interpretation of the obtained data reviled the mixture of oligopeptides and polypeptides with the mass range 500-7000 Dalton. See Figure 1.

### Preparation of protein hydrolysate from bovine albumin

This example is not according to the invention and is present for illustration purposes only. 100 mg of bovine albumin suspended in 1,5 ml of water together with 4 mg of **IP1** were stirred at 140°C for 1h. After reaction, the mixture was cooled to room temperature, filtered and the liquid phase was subjected for QTOF-ESI(+) analysis.

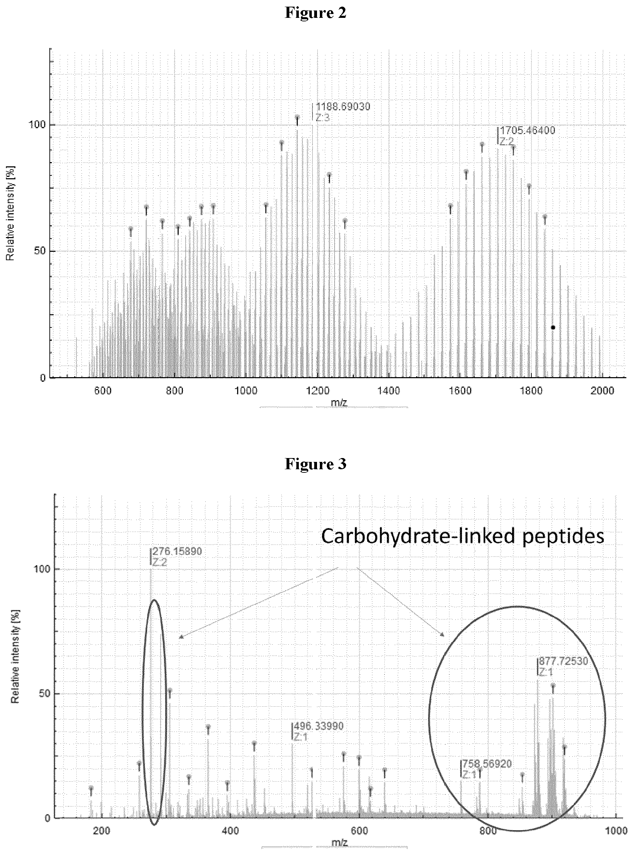

The interpretation of the obtained data reviled the mixture of oligopeptides and polypeptides with the mass range 500-8000 Da. See Figure 2.

### Preparation of protein hydrolysate from spent barley

This example is not according to the invention and is present for illustration purposes only. 200 g of spent barley suspended in 1,5 L of water together with 3 g of **IP1** were stirred at 140°C for 1h. After reaction, the mixture was cooled to room temperature, filtered, and the liquid phase was subjected for QTOF-ESI(+) analysis.

The interpretation of the obtained data reviled the presence of peptides linked to carbohydrates. See Figure 3.

### Preparation of protein hydrolysate from yeast slurry

67 g of yeast slurry (dry bases) suspended in 0,666 L of water together with 0,22 g of **IP3** were stirred at 145 °C for 1,5 h. After reaction, the mixture was cooled to room temperature, filtered and the liquid phase was dried using spray drier. The obtained dried product was analysed for protein content using Kjeldahl's method and 45 wt% as hydrolysed protein was measured.

### Example of physicochemical improvement of the proteins

Okara has a high nutritive value due to its high-quality protein (15.2-33.4%), fat (8.3-10.9%), carbohydrates and fibre (30-58%). Due to the technological process, the okara residue contains predominantly insoluble or non-extractable proteins. The okara protein isolates contain all of the essential amino acids and have a protein efficiency index that is even higher than that of soymilk (2.71 vs. 2.11) and tofu, but with low water solubility. It has also been found that the protein fraction of okara is able to withstand complete digestion by the gastrointestinal enzymes, pepsin and pancreatin (the latter of which mainly consists of trypsin, amylopsin and steapsin). However, the unwanted flavour of okara, called the "beany" flavour in their research, was a major challenge for its application and production of okara-based food products.

To address such challenge, a hydrolysis treatment may be applied resulting in an increase of the soluble fibre and soluble protein content, and thus improve nutritional quality and processing properties.

### Preparation of okara hydrolysate I:

10 g of okara (dry bases) suspended in 100 ml of water together with ionic polymer catalyst IP3 were stirred at 120°C for 1 h. After reaction, the mixture was cooled to room temperature, filtered and the liquid phase was dried using spray drier.

### Preparation of okara hydrolysate II:

10 g of okara (dry bases) suspended in 100 ml of water together with ionic polymer catalyst **IP3** were stirred at 130°C for 1 h. After reaction, the mixture was cooled to room temperature, filtered and the liquid phase was dried using spray drier.

| Main analysed components, g/100 g | Okara feedstock | Okara hydrolysate I | Okara hydrolysate II |
|---|---|---|---|
| **glucose** | 2,3 | 3,6 | 2,2 |
| **xylose** | 7,2 | 2,9 | 2,9 |
| **galactose** | 13,2 | 14,8 | 18,6 |
| **arabinose** | 5,8 | 9,3 | 9,6 |
| **mannose** | 5,7 | 6,8 | 4,5 |

| | | | |
|---|---|---|---|
| total | 34,2 | 37,4 | 37,8 |
| protein hydrolysate | 29 | 19.5 | 21 |

### Example of the beneficial effect on specific bacteria growth

This example is not according to the invention and is present for illustration purposes only.

Macronutrients and micronutrients can modulate the microbiota. The overall balance between the macronutrients such as protein, carbohydrate and fat is known to influence the composition and functional potential of the gut microbiota. The efficacy of spent barley hydrolysate (the composition Prembion comprising the protein hydrolysate of the invention) was compared to Glucose, a commercial XOS and FOS products and was tested measuring the growth curves of a *Bifidobacterium X* and *Lactobacillus X* bacterial strains, which activity has been linked to improvements in animal and human gut health.

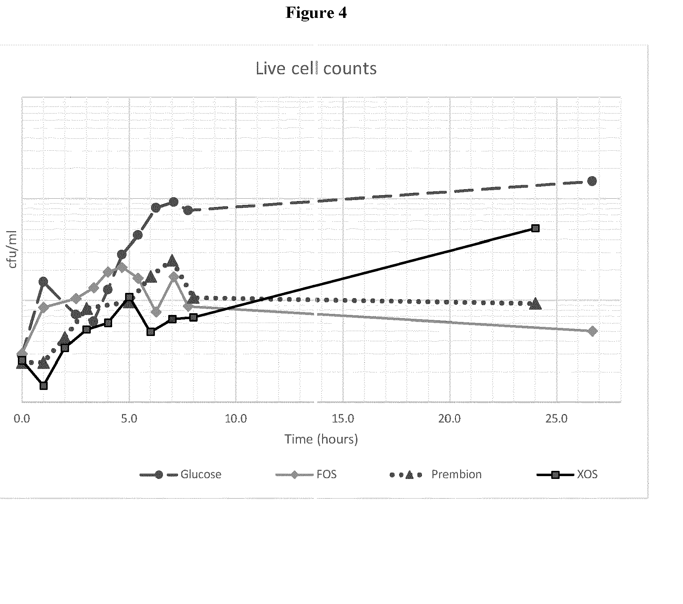

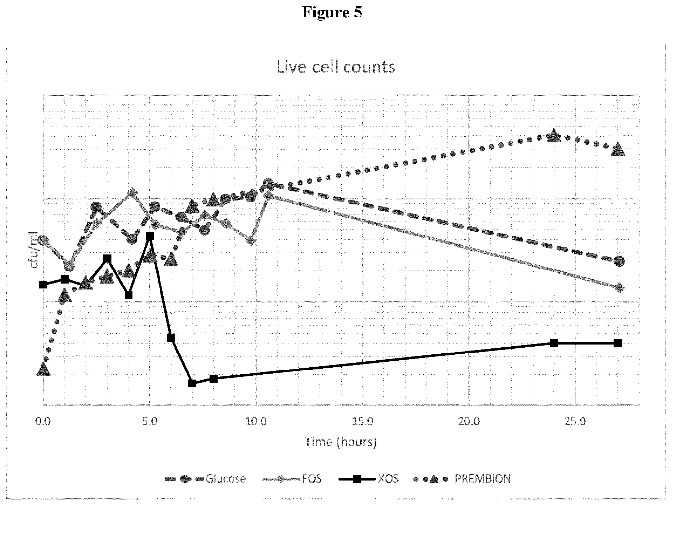

Figures 4 and 5 shows how two different strains perform *in vitro* when media is supplemented with different nutrient sources. When compared to pure FOS and XOS, a complex product derived from spent barley and containing hydrolysed protein demonstrated superior preferential growth of *Bifidobacterium* strain (Figure 5) and comparative growth of *Lactobacillus* strain (Figure 4).

## Claims

1. A method for hydrolysing proteins and/or protein-containing feedstock into a protein hydrolysate, the method comprising the steps of:
a) providing protein and/or protein-containing feedstock;
b) optionally determining protein content in the protein-containing feedstock;
c) optionally pre-treating the protein-containing feedstock;
d) optionally isolating proteins from the protein-containing feedstock to form a protein concentrate;
e) contacting the protein, the protein concentrate and/or the protein-containing feedstock with a catalyst to form a reaction mixture, wherein the catalyst is an ionic polymer, the ionic polymer network, a solid-supported ionic polymers and/or a polymer membrane incorporating ionic polymers;
f) degrading the protein, the protein concentrate and/or the protein-containing feedstock in the reaction mixture to produce a liquid phase and a solid phase, wherein the liquid phase includes the protein hydrolysate, and the solid phase includes residual materials;
g) isolating at least a portion of the liquid phase from the solid phase; and
h) recovering the protein hydrolysate from the isolated liquid phase,
wherein the ionic polymer (IP) is 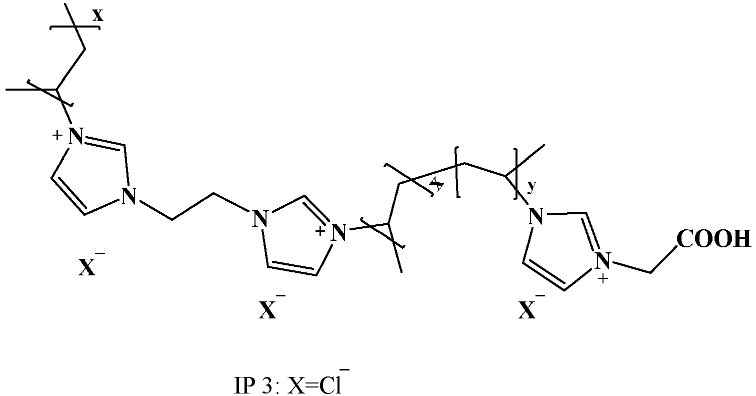
wherein x and y are integers each independently selected within the range 1 to 1000;
wherein the ionic polymer network comprises cross-linked the one or more ionic polymers (IP);
wherein the solid support has at least one surface comprising the one or more ionic polymers (IP) or the ionic polymer network;
wherein the polymer membrane incorporates the one or more ionic polymers (IP) or the ionic polymer network.

2. A protein hydrolysate obtained by the method of claim 1.

3. Use of the protein hydrolysate of claim 2 in the manufacturing of food products, animal feed products and cosmetic products.

4. A food product comprising an edible material and the protein hydrolysate of claim 2.

## Patentansprüche

1. Verfahren zur Hydrolysierung von Proteinen und/oder proteinhaltigem Ausgangsmaterial zu einem Proteinhydrolysat, wobei das Verfahren die folgenden Schritte umfasst:
a) Bereitstellen von Protein und/oder proteinhaltigem Ausgangsmaterial;
b) gegebenenfalls Bestimmen des Proteingehalts in dem proteinhaltigen Ausgangsmaterial;
c) gegebenenfalls Vorbehandeln des proteinhaltigen Ausgangsmaterials;
d) gegebenenfalls Isolieren von Proteinen aus dem proteinhaltigen Ausgangsmaterial, um ein Proteinkonzentrat zu bilden;
e) In-Kontakt-bringen des Proteins, des Proteinkonzentrats und/oder des proteinhaltigen Ausgangsmaterials mit einem Katalysator, um ein Reaktionsgemisch zu bilden, wobei es sich bei dem Katalysator um ein ionisches Polymer, das ionische Polymernetzwerk, ein ionisches Polymer mit festem Träger und/oder eine ionische Polymere enthaltende Polymermembran handelt;
f) Abbauen des Proteins, des Proteinkonzentrats und/oder des proteinhaltigen Ausgangsmaterials in dem Reaktionsgemisch, um eine flüssige Phase und eine feste Phase zu erzeugen, wobei die flüssige Phase das Proteinhydrolysat aufweist und die feste Phase Restmaterialien aufweist;
g) Isolieren zumindest eines Teils der flüssigen Phase aus der festen Phase; und
h) Gewinnen des Proteinhydrolysats aus der isolierten flüssigen Phase,
wobei es sich bei dem ionischen Polymer (IP) um 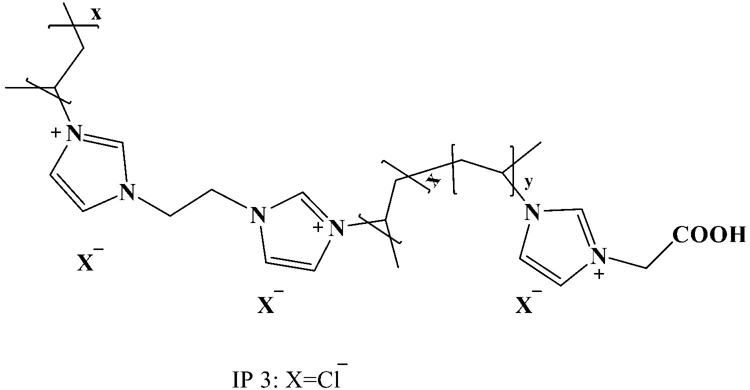
handelt, wobei x und y ganze Zahlen sind, die jeweils unabhängig innerhalb des Bereichs von 1 bis 1000 ausgewählt werden;
wobei das ionische Polymernetzwerk das eine oder die mehreren ionischen Polymere (IP) vernetzt umfasst;
wobei der feste Träger mindestens eine Oberfläche aufweist, die ein oder mehrere ionische Polymere (IP) oder das ionische Polymernetzwerk umfasst;
wobei die Polymermembran das eine oder die mehreren ionische Polymere (IP) oder das ionische Polymernetzwerk enthält.

2. Proteinhydrolysat, das durch das Verfahren nach Anspruch 1 erhalten wird.

3. Verwendung des Proteinhydrolysats nach Anspruch 2 bei der Herstellung von Nahrungsmittelprodukten, Tierfutterprodukten und Kosmetikprodukten.

4. Nahrungsmittelprodukt, umfassend essbares Material und das Proteinhydrolysat nach Anspruch 2.

## Revendications

1. Procédé d'hydrolyse de protéines et/ou d'une matière première contenant des protéines en un hydrolysat de protéines, le procédé comprenant les étapes suivantes :
a) fournir une protéine et/ou une matière première contenant des protéines ;
b) déterminer éventuellement la teneur en protéines de la matière première contenant des protéines ;
c) traiter éventuellement au préalable la matière première contenant des protéines ;
d) isoler éventuellement les protéines de la matière première contenant des protéines pour former un concentré de protéines ;
e) mettre en contact la protéine, le concentré de protéines et/ou la matière première contenant des protéines avec un catalyseur pour former un mélange réactionnel, dans lequel le catalyseur est un polymère ionique, le réseau de polymères ioniques, des polymères ioniques sur support solide et/ou une membrane polymère incorporant des polymères ioniques ;
f) dégrader la protéine, le concentré de protéines et/ou la matière première contenant des protéines dans le mélange réactionnel pour produire une phase liquide et une phase solide, la phase liquide comprenant l'hydrolysat de protéines et la phase solide comprenant des matières résiduelles ;
g) isoler au moins une partie de la phase liquide de la phase solide ; et
h) récupérer l'hydrolysat de protéines à partir de la phase liquide isolée,
dans lequel le polymère ionique (IP) est 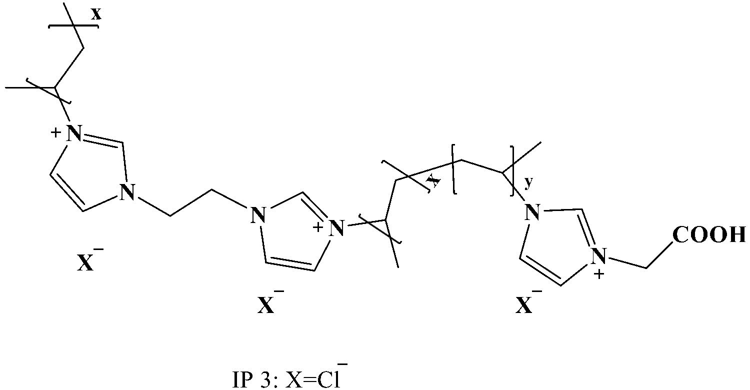
où x et y sont des nombres entiers choisis chacun indépendamment dans la plage de 1 à 1 000 ;
dans lequel le réseau de polymères ioniques comprend un ou plusieurs polymères ioniques (IP) réticulés ;
dans lequel le support solide présente au moins une surface comprenant un ou plusieurs polymères ioniques (IP) ou le réseau de polymères ioniques ;
dans lequel la membrane polymère incorpore un ou plusieurs polymères ioniques (IP) ou le réseau de polymères ioniques.

2. Hydrolysat de protéines obtenu par le procédé selon la revendication 1.

3. Utilisation de l'hydrolysat de protéines selon la revendication 2 dans la fabrication de produits alimentaires, d'aliments pour animaux et de produits cosmétiques.

4. Produit alimentaire comprenant une matière comestible et l'hydrolysat de protéines selon la revendication 2.
